# EUROPEAN PATENT SPECIFICATION

(11) **EP 4 615 368 B1**
(45) Date of publication and mention of the grant of the patent: **02.12.2026**
(21) Application number: 23818621.7
(22) Date of filing: 08.11.2023
(51) Int. Cl.: A61F 2/24

(54) **COMMISSURE ASSEMBLY FOR PROSTHETIC HEART VALVE**
KOMMISSURANORDNUNG FÜR EINE HERZKLAPPENPROTHESE
ENSEMBLE COMMISSURE POUR VALVE CARDIAQUE PROTHÉTIQUE

(30) Priority: 09.11.2022 US 202263423898 P
(43) Date of publication of application: 17.09.2025
(73) Proprietor: Edwards Lifesciences Corporation, Irvine, CA 92614 (US)
(72) Inventor: DALBOW, Brendan, Michael, Irvine, CA 92614 (US); SHITRIT, Roy, 3079892 Caesarea (IL); LEVI, Tamir, S., 3079892 Caesarea (IL)
(74) Representative: Eisenführ Speiser
(86) International application number: PCT/US2023/079068
(87) International publication number: WO 2024/102806

(56) References cited:
- WO-A1-2021/178317
- WO-A1-2022/026351
- AU-B2- 2019 272 012

## Description

### CROSS REFERENCE TO RELATED APPLICATION

This application claims the benefit of U.S. Application No. 63/423,898, filed on November 9, 2022.

### FIELD

The present disclosure relates to prosthetic heart valves, and to methods and assemblies for forming leaflet assemblies and attaching the leaflet assemblies to the frame of such prosthetic heart valves.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic heart valve reaches the implantation site in the heart. The prosthetic heart valve is then expanded to its functional size, for example, by inflating a balloon on which the prosthetic valve is mounted, actuating a mechanical actuator that applies an expansion force to the prosthetic heart valve, or by deploying the prosthetic heart valve from a sheath of the delivery apparatus so that the prosthetic heart valve can self-expand to its functional size.

An important design parameter of a transcatheter heart valve is the diameter of the folded or crimped profile. The diameter of the crimped profile is important because it directly influences the physician's ability to advance the transcatheter heart valve through the femoral artery or vein. More particularly, a smaller profile allows for treatment of a wider population of patients, with enhanced safety.

Most expandable, transcatheter heart valves comprise a valvular structure disposed within the frame and attached to the frame via a plurality of commissures. Commissures can be formed by connecting adjacent leaflets of the valvular structure to one another and to the frame. However, such assembly techniques can take significant time and effort, thereby increasing production time and costs.

Accordingly, a need exists for simplifying an assembly process of the prosthetic heart valve, including simplifying the process for attaching the valvular structure to the frame of the prosthetic heart valve.

AU 2019 272 012 B2 relates to an assembly for implanting a balloon-expandable prosthetic aortic valve in a patient's body, the assembly comprising: a delivery apparatus comprising an elongated shaft having an inflatable balloon, and a radially expandable prosthetic aortic valve adapted to be mounted on the shaft in a radially collapsed configuration for delivery into the body, the prosthetic aortic valve comprising: a balloon-expandable annular frame having an inflow end portion and an outflow end portion; a leaflet structure positioned within the frame, the leaflet structure comprising a plurality of commissure portions; and an annular inner skirt positioned along an inner surface of the frame, the inner skirt being secured to the inside of the frame; wherein the frame comprises a plurality of angularly spaced commissure windows each comprising an enclosed opening between first and second axially oriented side struts, wherein the plurality of commissure portions extend outwardly through respective commissure windows; wherein the prosthetic aortic valve further comprises an annular outer skirt positioned around an outer surface of the frame, the outer skirt comprising an inflow edge secured to an inflow edge of the inner skirt at the inflow end portion of the frame, an outflow edge secured to the frame at a second location, and an intermediate portion between the inflow edge and the outflow edge; and wherein when the prosthetic aortic valve is in the expanded configuration, the intermediate portion of the outer skirt comprises slack in the axial direction between the inflow edge of the outer skirt and the outflow edge of the outer skirt, and when the prosthetic aortic valve is collapsed to the collapsed configuration, the axial distance between the inflow edge of the outer skirt and the outflow edge of the outer skirt increases, reducing the slack in the outer skirt in the axial direction, wherein the outflow edge of the outer skirt comprises a plurality of alternating projections and notches, the projections being secured to the frame at the second location, the outer skirt being unsecured to the frame at the notches.

WO 2022/026351 A1 provides an implantable prosthetic device that includes a frame movable between a radially compressed configuration and a radially expanded configuration, and a valvular structure. The frame can have an inflow orifice, an outflow orifice, and one or more commissure windows. The valvular structure can include a plurality of leaflets, each leaflet having a main body with an inflow edge, an outflow edge, and a pair of opposing tabs. Each tab can be paired with an adjacent tab of an adjacent leaflet to form a commissure tab assembly, and each commissure tab assembly being coupled to a respective commissure window. Each tab can extend from the main body at an angle such that a radially outer edge of the tab corresponds to a draft angle of the frame.

WO 2021/178317 A1 describes a prosthetic heart valve and associated methods for assembling a prosthetic heart valve including a plurality of leaflets. In one described example, a prosthetic heart valve includes a frame; a plurality of commissure support elements, each connected the frame and including two axially-extending members spaced apart from one another to form a window; and a plurality of leaflets, each leaflet comprising a body and two opposing commissure tabs, each commissure tab arranged adjacent to another commissure tab to form a pair of commissure tabs that are disposed in a commissure support element to form a commissure. Each commissure tab includes a series of overlapping layers that include a first set of two overlapping layers that extends through the window and a second set of two overlapping layers that extends away from the first set of two overlapping layers and over an outer side of a corresponding axially-extending member.

### SUMMARY

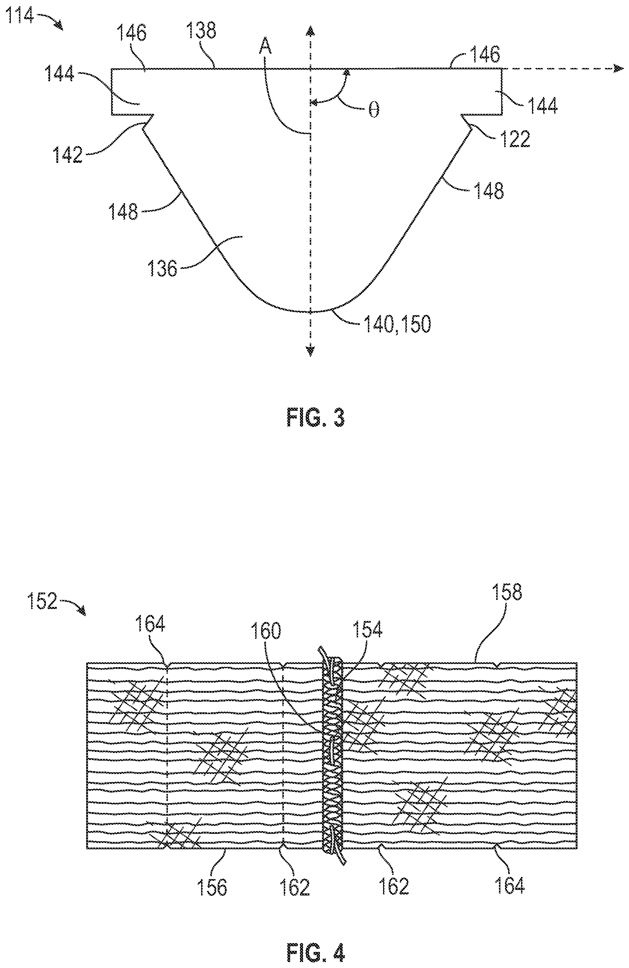

The claimed invention is defined in independent claim 1 and relates to an implantable prosthetic device. Some preferred configurations of the claimed invention are defined in the dependent claims. Certain aspects and/or particularly preferred configurations of the claimed invention are discussed hereafter for example in conjunction with Figs. 3-7. Also described herein are related aspects, examples, embodiments and arrangements useful for understanding the claimed invention, and which do not necessarily constitute embodiments of the claimed invention. Features recited in the independent claim without any indication of being optional are to be understood as obligatory, irrespective whether or not conditional language is used in the following description. The subject-matter for which protection is sought is defined by the claims.

Described herein are prosthetic heart valves, delivery apparatus, and methods for implanting prosthetic heart valves. The disclosed prosthetic heart valves, delivery apparatus, and methods can, for example, provide commissure configurations that minimize the amount of material in the commissure, thus helping reduce the overall bulk and width of the commissure, which can be important for retaining coronary access and for minimizing the crimp profile of the prosthetic valve. As such, the devices and methods disclosed herein can, among other things, overcome one or more of the deficiencies of typical prosthetic heart valves and their delivery apparatus.

A prosthetic heart valve can comprise a frame and a valve structure coupled to the frame. In addition to these components, a prosthetic heart valve can further comprise one or more of the components disclosed herein.

In some examples, the prosthetic valve can comprise commissures wherein the overall width of the commissure is equal to or less than the collective width of one or more axially extending struts of the frame and a commissure window of the frame.

In some examples, the commissure tabs of two adjacent leaflets of the valve structure can be coupled to a flexible connector via one or more primary sutures.

In some examples, the prosthetic valve can comprise commissures having first and second secondary sutures that couple the commissure to a commissure window of the frame, each secondary suture extending through a radially outer layer of the flexible connector, a respective commissure tab, a radially inner layer of the flexible connector, and a free edge portion of the flexible connector.

In some examples, the prosthetic valve can comprise commissures having a secondary suture that couples the commissure to a commissure window of the frame, each secondary suture extending in a figure-eight pattern.

In some examples, the figure-eight pattern extends through a radially outer layer of a flexible connector, a first commissure tab, a first radially inner layer of the flexible connector, a first free edge portion of the flexible connector, a second commissure tab, a second radially inner layer of the flexible connector, and a second free edge portion of the flexible connector.

In some examples, the figure-eight pattern is a first figure-eight pattern, and the secondary suture is sewn in a plurality of figure-eight patterns extending axially along a length of the commissure.

In some examples, the prosthetic valve can comprise commissures having a flexible connector, the flexible connector including an opening that extends through a thickness of the flexible connector, the opening sized to allow a wedge member to be inserted through the opening.

In some examples, each commissure can comprise a wedge member.

In some examples, the first and second secondary sutures extend through the wedge member.

In some examples, the one or more primary sutures can be disposed adjacent a radially inner facing surface of the frame.

In some examples, an implantable prosthetic device, can comprise a radially expandable and compressible annular frame comprising an inflow end portion and an outflow end portion, the frame comprising one or more axially-extending struts that defining one or more commissure windows. The prosthetic device can further comprise a valvular structure comprising a plurality of leaflets arranged within the frame, each leaflet comprising a main body and one or more commissure tabs, and a commissure comprising a flexible connector and the commissure tabs of two adjacent leaflets coupled to the flexible connector via one or more primary sutures. The commissure can be secured to a respective commissure window via one or more secondary sutures, each secondary suture extending through a radially outer layer of the flexible connector, a respective commissure tab, a radially inner layer of the flexible connector, and a free edge portion of the flexible connector.

In some examples, an implantable prosthetic device, can comprise a radially expandable and compressible annular frame comprising an inflow end portion and an outflow end portion, the frame comprising one or more axially-extending struts that defining one or more commissure windows. The prosthetic device can further comprise a valvular structure comprising a plurality of leaflets arranged within the frame, each leaflet comprising a main body and opposing commissure tabs, a flexible connector comprising a main body, a first free edge portion, and a second free edge portion, and a commissure comprising the flexible connector, a wedge member disposed on a radially inner surface of the flexible connector, and first and second commissure tabs of two adjacent leaflets coupled to the flexible connector via first and second primary sutures. The commissure can be secured to a respective commissure window via first and second secondary sutures, the first secondary suture extending through a radially outer layer of the flexible connector, the wedge member, the first commissure tab, a first radially inner layer of the flexible connector, and the first free edge portion, the second secondary suture extending through the radially outer layer of the flexible connector, the wedge member, the second commissure tab, a second radially inner layer of the flexible connector, and the second free edge portion.

In some examples, an implantable prosthetic device can comprise a radially expandable and compressible annular frame comprising an inflow end portion and an outflow end portion, the frame comprising one or more axially-extending struts that defining one or more commissure windows. The prosthetic device can further comprise a valvular structure comprising a plurality of leaflets arranged within the frame, each leaflet comprising a main body and opposing commissure tabs, a flexible connector comprising a main body, a first free edge portion, and a second free edge portion, and a commissure comprising the flexible connector and first and second commissure tabs of two adjacent leaflets coupled to the flexible connector via first and second primary sutures. The commissure can be secured to a respective commissure window via a secondary suture sewn in a figure-eight pattern.

In some examples, an implantable prosthetic device, can comprise a radially expandable and compressible annular frame comprising an inflow end portion and an outflow end portion, the frame comprising one or more axially extending struts that defining one or more commissure windows. The prosthetic device can further comprise a valvular structure comprising a plurality of leaflets arranged within the frame, each leaflet comprising a main body and opposing commissure tabs, a flexible connector comprising a main body, a first free edge portion, a second free edge portion, and an opening extending through a thickness of the main body, and a commissure comprising the flexible connector and first and second commissure tabs of two adjacent leaflets coupled to the flexible connector via first and second primary sutures, the commissure further comprising a wedge member disposed on a radially inner surface of the flexible connector such that it is visible through the opening.

In some examples, a method of assembling a valvular structure can comprise coupling one or more commissure tabs of one or more adjacent leaflets to a flexible connector using one or more primary sutures, and advancing a portion of the flexible connector and the one or more commissure tabs through a commissure window in a radially expandable and compressible annular frame, the commissure window defined by one or more axially extending struts. The method can further comprise folding the one or more commissure tabs such that radially inner facing surfaces of the one or more commissure tabs are adjacent a radially outer facing surface of the frame, wrapping first and second free end portions of the flexible connector around the one or more axially extending struts, and securing the commissure to the frame via one or more secondary sutures, each secondary suture extending through a radially outer layer of the flexible connector, a respective commissure tab, a radially inner layer of the flexible connector, and a free edge portion of the flexible connector.

In some examples, a method of assembling a valvular structure can comprise coupling a first integral commissure tab of a first leaflet to a first side portion of a flexible connector using a first primary suture, coupling a second integral commissure tab of a second leaflet to a second side portion of the flexible connector using a second primary suture, and advancing a portion of the first and second commissure tabs and the flexible connector through a commissure window in a radially expandable and compressible annular frame, the commissure window defined by one or more axially extending struts. The method can further comprise folding the first and second commissure tabs circumferentially away from one another such that radially inner facing surfaces of the first and second commissure tabs are adjacent a radially outer facing surface of the frame, wrapping first and second free end portions of the flexible connector around the one or more axially extending struts, and securing the commissure to the frame via a first secondary suture extending through a radially outer layer of the flexible connector, the wedge member, the first commissure tab, a first radially inner layer of the flexible connector, and the first free edge portion, and a second secondary suture extending through the radially outer layer of the flexible connector, the wedge member, the second commissure tab, a second radially inner layer of the flexible connector, and the second free edge portion.

In some examples, a method of assembling a valvular structure can comprise coupling a first integral commissure tab of a first leaflet to a first side portion of a flexible connector using a first primary suture, coupling a second integral commissure tab of a second leaflet to a second side portion of the flexible connector using a second primary suture, and advancing a portion of the first and second commissure tabs and the flexible connector through a commissure window in a radially expandable and compressible annular frame, the commissure window defined by one or more axially extending struts. The method can further comprise folding the first and second commissure tabs circumferentially away from one another such that radially inner facing surfaces of the first and second commissure tabs are adjacent a radially outer facing surface of the frame, wrapping first and second free end portions of the flexible connector around the one or more axially extending struts, and securing the commissure to the frame via a secondary suture sewn in a figure-eight pattern.

In some examples, a method comprises sterilizing any of the above described devices.

The various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the disclosure will become more apparent from the following detailed description, claims, and accompanying figures.

### BRIEF DESCRIPTION OF THE DRAWINGS

FIG. 1 is a side elevation view of a prosthetic heart valve, according to one example.
FIG. 2 is a perspective view of the frame of the prosthetic heart valve of FIG. 1.
FIG. 3 is a plan view of a leaflet of the prosthetic heart valve of FIG. 1, shown in a flattened configuration.
FIG. 4 is a plan view of an example of a flexible connector, shown in a flattened configuration.
FIGS. 5-7 are cross-sectional views illustrating steps for assembling a commissure and coupling the commissure to the frame of a prosthetic heart valve.
FIG. 8 is a cross-sectional view of a commissure coupled to the frame of a prosthetic heart valve, according to one example.
FIG. 9 is a side elevational view of the commissure of FIG. 8 shown coupled to the frame of a prosthetic heart valve.
FIG. 10 is a plan view of an example of a flexible connector, shown in a flattened configuration.

### DETAILED DESCRIPTION

### General Considerations

For purposes of this description, certain aspects, advantages, and novel features of the examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

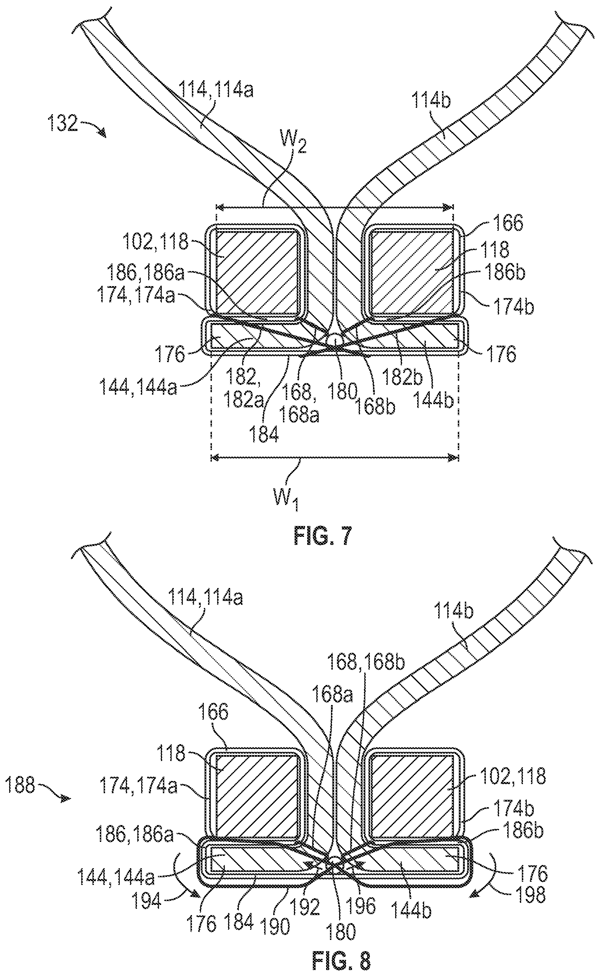

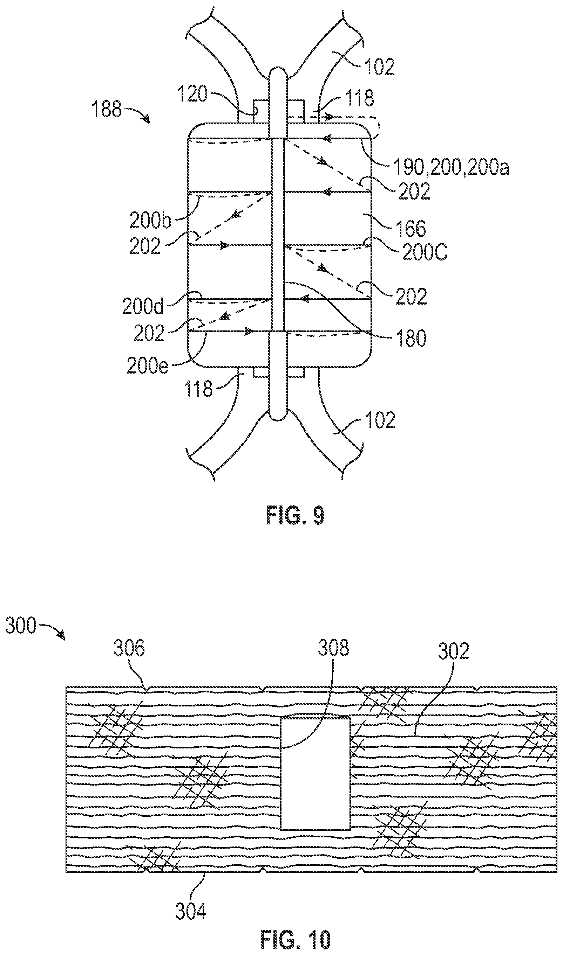

All features described herein are independent of one another and, except where structurally impossible, can be used in combination with any other feature described herein. For example, a flexible connector 300 as shown in FIG. 10 or a flexible connector 152 as shown in FIG. 4 can be used in combination with any of the commissures 132, 188 described herein. In some examples, secondary figure-eight sutures 190 as shown in FIGS. 8-9 can be used in combination with secondary sutures 182 shown in FIG. 7.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

In some examples, values, procedures, or apparatus may be referred to as "lowest," "best," "minimum," or the like. It will be appreciated that such descriptions are intended to indicate that a selection among many alternatives can be made, and such selections need not be better, smaller, or otherwise preferable to other selections.

In the description, certain terms may be used such as "up," "down," "upper," "lower," "horizontal," "vertical," "left," "right," and the like. These terms are used, where applicable, to provide some clarity of description when dealing with relative relationships. But, these terms are not intended to imply absolute relationships, positions, and/or orientations. For example, with respect to an object, an "upper" surface can become a "lower" surface simply by turning the object over. Nevertheless, it is still the same object.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

### Examples of the Disclosed Technology

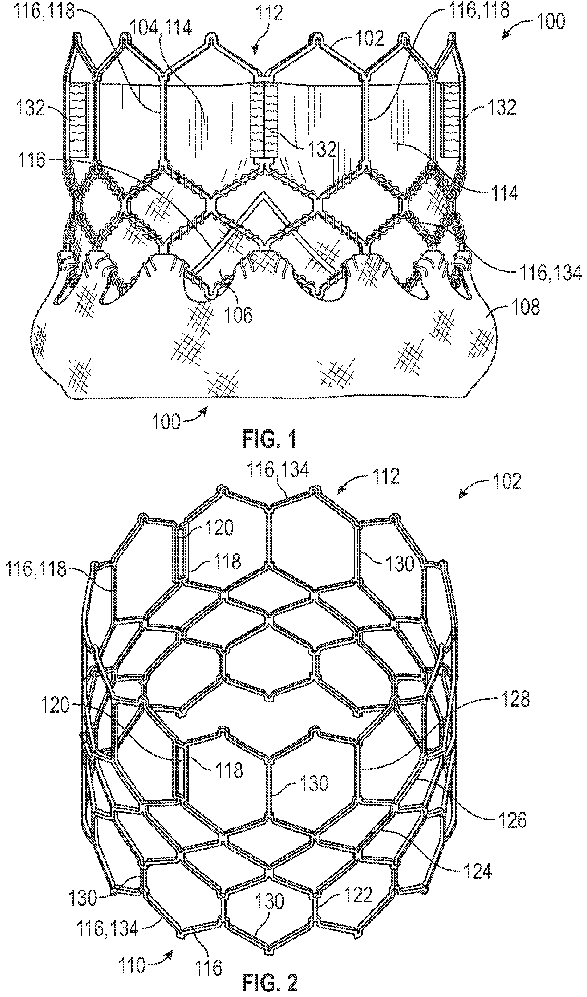

FIGS. 1 shows a prosthetic heart valve 100, according to one example. The illustrated valve is adapted to be implanted in the native aortic annulus, although in other examples it can be adapted to be implanted in the other native annuluses of the heart. The valve 100 can generally comprise a stent or frame 102, a valvular or leaflet structure 104, an inner skirt 106, and an outer skirt 108. The prosthetic heart valve can have an inflow end portion 110 and an outflow end portion 112.

The valvular structure 104 can comprise three leaflets 114 which can be arranged to collapse in a tricuspid arrangement. The lower edge of leaflet structure 104 desirably has an undulating, curved scalloped shape (suture line 116 shown in FIG. 1 tracks the scalloped shape of the leaflet structure). By forming the leaflets with this scalloped geometry, stresses on the leaflets are reduced, which in turn improves durability of the valve. Moreover, by virtue of the scalloped shape, folds and ripples at the belly of each leaflet (the central region of each leaflet), which can cause early calcification in those areas, can be eliminated or at least minimized. The scalloped geometry also reduces the amount of tissue material used to form leaflet structure, thereby allowing a smaller, more even crimped profile at the inflow end of the valve. The leaflets 114 can be formed of pericardial tissue (e.g., bovine pericardial tissue), biocompatible synthetic materials, or various other suitable natural or synthetic materials as known in the art and described at least in U.S. Patent No. 6,730,118.

FIG. 2 illustrates the frame 102 with the other components removed for purposes of illustration. The frame 102 can comprise a plurality of struts 116, including a plurality of axially-extending struts 118, one or more of which can define commissure windows 120 (for example, three in the illustrated example). The commissure windows 120 are adapted to mount the commissures of the valvular structure 104 to the frame, as described in greater detail below. The frame 102 can be made of any of various suitable plastically-expandable materials (*e.g.,* stainless steel, etc.) or self-expanding materials (*e.g.,* Nitinol) as known in the art. When constructed of a plastically-expandable material, the frame 102 (and thus the valve 100) can be crimped to a radially compressed state on a delivery catheter and then expanded inside a patient by an inflatable balloon or equivalent expansion mechanism. When constructed of a self-expandable material, the frame 102 (and thus the valve 100) can be crimped to a radially compressed state and restrained in the compressed state by insertion into a sheath or equivalent mechanism of a delivery catheter. Once inside the body, the valve 100 can be advanced from the delivery sheath, which allows the valve to expand to its functional size.

Suitable plastically-expandable materials that can be used to form the frames disclosed herein (for example, the frame 102) include, metal alloys, polymers, or combinations thereof. Example metal alloys can comprise one or more of the following: nickel, cobalt, chromium, molybdenum, titanium, or other biocompatible metal. In some examples, the frame 102 can comprise stainless steel. In some examples, the frame 102 can comprise cobalt-chromium. In some examples, the frame 102 can comprise nickel-cobalt-chromium. In some examples, the frame 102 comprises a nickel-cobalt-chromium-molybdenum alloy, such as MP35N^{™} (tradename of SPS Technologies), which is equivalent to UNS R30035 (covered by ASTM F562-02). MP35N^{™}/UNS R30035 comprises 35% nickel, 35% cobalt, 20% chromium, and 10% molybdenum, by weight. In some examples, when MP35N is used as the frame material, less material is needed to achieve the same or better performance in radial and crush force resistance, fatigue resistances, and corrosion resistance. Moreover, since less material is required, the crimped profile of the frame can be reduced, thereby providing a lower profile valve assembly for percutaneous delivery to the treatment location in the body.

The frame 102 can comprise a plurality of struts 116 arranged to define one or more circumferentially extending rows of cells. In the illustrated example, the frame 102 can comprise four circumferentially extending rows of cells 130, namely, first row 122, second row 124, third row 126, and fourth row 128. However, in other examples, the frame 102 can have a greater or fewer number of rows. In some examples, the cells 130 in one or more rows of cells can have a different shape relative to the remaining cells. For example, in the illustrated example, the fourth row of cells 128 (disposed adjacent the outflow end portion 112 of the frame 102) can have a relatively larger open cell area compared to the cells of rows 126, 124, and/or 122. Accordingly, the cells 130 in the fourth row 128 can be referred to as "larger" or "elongated" cells. The elongated cells are relatively large and are sized to allow portions of the valvular structure 104 to protrude, or bulge, into and/or through the cells 130 when the frame 120 is crimped in order to minimize the crimping profile. In some examples, the cells 130 in the first row of cells 122 can also have a relatively larger open cell area compared to the cells of rows 124 and/or 126.

As mentioned previously and shown in FIG. 1, the prosthetic valve 100 can also include a valvular structure 104, which is coupled to and supported by the frame 102. The valvular structure 104 can include, for example, one or more leaflets 114. The leaflets 114 can be secured to one another at their adjacent sides to form commissures 132, each of which can be secured to a respective commissure window 120 of the frame 102. The axially-extending struts 118 which define the commissure windows 120 can be secured at their upper and lower ends to angled struts 134 to provide a robust configuration that enhances fatigue resistance under cyclic loading of the valve. In the illustrated example, the commissure windows 120 can have an elongated rectangular shape. However, in other examples, the commissure windows can have any of various shapes, including but not limited to, square, oval, square oval, triangular, trapezoidal, etc.

Further details of the frame and leaflet structure can be found, at least, in U.S. Patent 9,393,110.

FIG. 3 illustrates a leaflet 114 of the valvular structure 104 according to one example. The leaflet 114 can comprise a main body 136, an upper edge portion 138 (also referred to as the coaptation edge or free edge), a lower edge portion 140 (also referred to as the cusp edge or scallop edge) which terminates at openings/recesses/gaps 142 that extend laterally into the main body 136 of the leaflet, and laterally projecting integral tabs 144 (also referred to as commissure tabs), which are spaced apart from the lower edge portion 140 by the gaps 142. In some examples, such as the illustrated example, the tabs 144 are formed integrally with the main body 136 of the leaflet. The tabs 144 can extend from the main body sch that an upper or outflow edge 146 of each tab is positioned at an angle Θ relative to a longitudinal axis A of the leaflet 114. In the illustrated example, the tabs 144 can extend from the main body 136 such that the angle Θ is a 90 degree angle. In other examples, the angle Θ can be any of various angles, for example, corresponding to (for example, being substantially parallel to) the draft angle of the frame of the prosthetic valve.

The leaflet 114 can have an overall V-shape. The sub-commissure portion of the leaflet 114 (that is, the portion below the tabs 144) includes two substantially straight edges 148 that extend from respective locations below the tabs 144 to a curved lower edge 150. The tapered profile of the sub-commissure portion of the leaflet reduces the amount of leaflet material in the lower half of the crimped valve to minimize the crimp diameter of that portion of the valve. Thus, if additional components are mounted to that portion of the valve, such as an outer skirt 108, the reduced profile of that portion of the valve can help offset or minimize the increase in diameter caused by the additional component. Additionally, the commissure tabs 144 are relatively short and require fewer sutures for forming the commissures of the leaflet structure than other known commissure designs, which better distributes and reduces the bulkiness of the leaflet material when the valve is crimped.

As mentioned, the leaflets 114 can be secured to one another at their adjacent sides to form commissures 132 of the valvular structure. A plurality of flexible connectors 152, also referred to as "commissure attachment members" or "posts" (one of which is shown in FIG. 4) can be used to interconnect pairs of adjacent tabs 144 and to mount the leaflets to the commissure window frame portions 120. In some examples, the flexible connectors 152 can be made from a piece of woven PET fabric, although other synthetic and/or natural materials can be used. In some examples, the flexible connector 152 can include a reinforcing member/wedge member 154 extending from the lower or inflow edge 156 to the upper or outflow edge 158 at the center of the connector 152. The wedge member 154 can comprise a non-metallic material, such as a rope, a piece of fabric (such as PET), and/or a piece of Ethibond 2-0 suture material, secured to the connector (for example, with a temporary suture 160). The wedge member 154 can help prevent rotational and radial movement of the leaflet tabs once they are secured to the commissure window frame portions 120. In some examples, the connector 152 can have one or more inner notches 162 and/or outer notches 164 formed along its upper and lower edges. The notches 162, 164 can be used to help align the flexible connector to the leaflets 114 and for folding during the commissure assembly process.

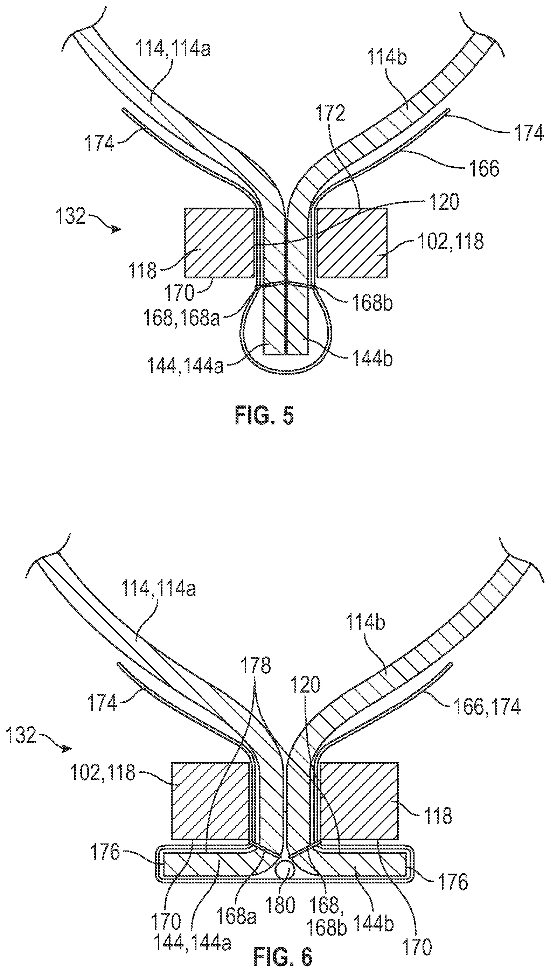

FIGS. 5-7 illustrate the formation of an exemplary commissure 132 for coupling a portion of the valvular structure 104 to the frame 102. To form each commissure 132, the tab portions 144a, 144b of two adjacent leaflets 114a, 114b can be coupled to a respective flexible connector 166, as shown in FIG. 5. The flexible connector 166 can be the same or similar to flexible connector 152 described previously. A first stitch/suture/suture line 168a can extend through the first tab portion 144a and the flexible connector 166, and a second stitch/suture/suture line 168b can extend through the second tab portion 144b and the flexible connector 166. The first and second suture lines 168a, 168b can collectively be referred to as the "primary suture lines" 168.

Thus coupled, the tabs 144a, 144b and flexible connector 166 can be inserted through the commissure opening 120 of the frame 120, as shown in FIG. 5. The free end portions 174 of the flexible connector 166 can remain radially inside of the frame 102. The tabs 144a, 144b can be advanced until the primary sutures 168 are positioned adjacent the radially outer surface 170 of the frame 102. In other examples, the primary sutures 168 can be positioned such that they are adjacent the radially inner surface 172 of the frame 102. Coupling the tabs 144a, 144b to the flexible connector 166 prior to insertion through the commissure window 120 can advantageously allow the tabs 144a, 144b to be pulled rather than pushed through the commissure window 120, which can prevent or mitigate damage to the tabs during this step of assembly.

Referring to FIG. 6, the edge portions 176 of the tabs 144a, 144b can be folded circumferentially outwardly such that a radially inner facing surface of the tabs 178 is positioned adjacent to the radially outer facing surface 170 of the frame 102. A wedge member 180 can be positioned on a radially inner surface of the flexible connector 166 between the first and second tabs 144a, 144b as shown in FIG. 6. In some examples, the wedge 180 can be inserted after the tabs 144 are folded circumferentially, however, in other examples, the wedge member 180 can be coupled to the flexible member 166 prior to coupling the flexible member to the tabs 144.

Referring now to FIG. 7, the first and second free end portions 174a, 174b of the flexible connector 166 can be folded radially outwardly such that they wrap around the struts 118 of the frame 102 that define the commissure window 120. A third stitch/suture/suture line 182a can extend through the first free end portion 174a, through the leaflet tab 144a, through the wedge member 180, and through one or more layers of the flexible connector 166. For example, in the illustrated example, the third suture 182 can extend through an outer layer 184 of the flexible connector 166, the wedge member 180, the first tab 144a, a first inner layer 186a of the flexible connector, and the first free end portion 174a of the flexible connector 166. A fourth stitch/suture/suture line 182b can extend through the second free end portion 174b, through the second leaflet tab 144b, through the wedge member 180, and through one or more layers of the flexible connector 166. For example, in the illustrated example, the fourth suture 182b can extend through the outer layer 184 of the flexible connector 166, the wedge member 180, the second tab 144b, a second inner layer 186b of the flexible connector 166, and the second free end portion 174b. The third and fourth suture lines can collectively be referred to as the "secondary suture lines" 182.

As shown in FIG. 7, the secondary suture lines 182 can cross over one another to form a X-shape. Such a configuration includes fewer assembly steps, facilitates a simplified commissure assembly process, and reduces the overall number of suture lines extending through the commissure 132 thus helping reduce the overall bulk and width of the commissure, which can be important for retaining coronary access and for minimizing the crimp profile of the prosthetic valve. The leaflet tabs 144 can be sized such that when folded they have a width W₁ that is substantially equal to or less than the collective width W₂ of the struts 118 that define the commissure window 120 and the commissure window 120 itself, and the commissure configuration 132 can use fewer sutures than previous configurations, thereby reducing the overall material present in the commissure. These features additionally help reduce the overall width of the commissure, which can advantageously minimize the overall profile of the valve, particularly when in the crimped configuration. In some examples, the commissures described herein can have an overall width of less than about 4 mm. For example, between about 1.5 mm and about 2.5 mm, between about 1.8 mm and about 2.3 mm, between about 2 mm and about 2.1 mm.

FIG. 8 illustrates an alternative suture configuration to the configuration illustrated in FIG. 7, resulting in an alternative commissure 188. The steps leading up to FIG. 8 are the same as those shown and described with respect to FIGS. 5-6. As shown in FIG. 8, in some examples, in lieu of third and fourth sutures 182a, 182b (the "secondary suture lines"), the commissure 188 can comprise a secondary suture line formed by suture 190. **In** some examples, the secondary suture line can comprise only a single suture.

The suture 190 can extend through the various components of the commissure in a 'figure-eight' pattern, such as shown in FIG. 8. That is, as illustrated by arrow 192, the suture 190 can extend through the outer layer 184 of the flexible connector 166, the wedge member 180, the first tab 144a, the first inner layer 186a of the flexible connector, and the first free end portion 174a. As illustrated by arrow 194, the suture 190 can then wrap around the edge portion 176 of the first tab 144a and then, as shown by arrow 196, extend through the outer layer 184 of the flexible connector and through the wedge member 180 (crossing over itself in an X-shape), then through the second tab 144b, the second inner layer 186b of the flexible connector 166, and the second free end portion 174b. The suture 190 can then wrap around the edge portion 176 of the second tab 144b, as shown by arrow 198.

Such a configuration advantageously secures the edge portions 176 of the tabs 144 while also simplifying the commissure assembly, and minimizing the amount of material in the commissure (one secondary suture line rather than two). Additionally, this configuration allows the commissure 188 to be tightened into its final configuration by pulling/tightening a single suture 190, rather than having to tighten various sutures.

As mentioned previously, the leaflet tabs 144 can be sized such that when folded against the radially outer facing surface of the frame they have a width that is substantially the same as the collective width of the struts 118 that define the commissure window 120 and the commissure window 120 itself, and the commissure configuration 188 can use fewer sutures than previous configurations, thereby reducing the overall material present in the commissure. These features additionally help reduce the overall width of the commissure which can advantageously minimize the overall profile of the valve, particularly when in the crimped configuration. In some examples, the commissures described herein can have an overall width of less than about 4 mm. For example, between about 1.5 mm and about 2.5 mm, between about 1.8 mm and about 2.3 mm, between about 2 mm and about 2.1 mm.

Referring to FIG. 9, in some examples, the "figure-eight" pattern of the secondary suture line 190 can continue in an axial direction along the length of the commissure 188 to form a plurality of figure-eight sutures 200. For example, once a first figure-eight 200a has been completed, the suture 190 can be sewn in an axial direction (e.g., toward the inflow end) before beginning a second figure-eight 200b. In some examples, such as shown in FIG. 9, the axially-extending portions 202 of the suture 190 can be disposed on alternating sides of the wedge member 180. In the illustrated example, the commissure 188 comprises a first figure-eight 200a, a second figure-eight 200b, a third figure-eight 200c, a fourth figure-eight 200d, and a fifth figure-eight 200e. In other examples, the commissure 188 can comprise one, two, three, four, five, six, seven, eight, nine, or ten figure-eight sutures.

FIG. 10 illustrates another example of a flexible connector 300, which can be used in lieu of or in addition to flexible connectors 152 or 166 described previously. Flexible connector 300 can be similar to flexible connectors 152 and 166 (e.g., having a main body 302 made from a piece of woven PET fabric, although other synthetic and/or natural materials can be used, an inflow edge 304, and an outflow edge 306), except that flexible connector 300 includes an optional aperture/window/opening 308 extending through a thickness of the flexible connector 300. The opening 308 can be configured to allow insertion of a wedge member, e.g., wedge member 180 described previously, through the opening 308. This can further simplify assembly of the commissure and can help ensure that the secondary suture lines 182/190 pass through the wedge member 180 during the assembly process.

Any of the systems, devices, apparatuses, etc. herein can be sterilized (for example, with heat/thermal, pressure, steam, radiation, and/or chemicals, etc.) to ensure they are safe for use with patients, and any of the methods herein can include sterilization of the associated system, device, apparatus, etc. as one of the steps of the method. Examples of heat/thermal sterilization include steam sterilization and autoclaving. Examples of radiation for use in sterilization include, without limitation, gamma radiation, ultra-violet radiation, and electron beam. Examples of chemicals for use in sterilization include, without limitation, ethylene oxide, hydrogen peroxide, peracetic acid, formaldehyde, and glutaraldehyde. Sterilization with hydrogen peroxide may be accomplished using hydrogen peroxide plasma, for example.

## Claims

1. An implantable prosthetic device, comprising:
a radially expandable and compressible annular frame (102) comprising an inflow end portion (110) and an outflow end portion (112), the frame (102) comprising one or more axially-extending struts (118) that define one or more commissure windows (120);
a valvular structure (104) comprising a plurality of leaflets (114) arranged within the frame (102), each leaflet (114) comprising a main body and one or more commissure tabs (144);
a commissure (132) comprising a flexible connector (152) and the commissure tabs (144) of two adjacent leaflets (114) coupled to the flexible connector (152) via one or more primary sutures (168); and
wherein the commissure (132) is secured to a respective commissure window (120) via one or more secondary sutures (182), each secondary suture (182) extending through a radially outer layer (184) of the flexible connector (152), a respective commissure tab (144), a radially inner layer (186) of the flexible connector (152), and a free edge portion (174) of the flexible connector (152); and
wherein:
an overall width (W1) of the commissure (132) is equal to or less than a collective width (W2) of the one or more axially extending struts (118) and the commissure window (120); or
the commissure tabs (144) are sized such that when folded against a radially outer facing surface of the frame (102) they have a width (W1) that is substantially equal to or less than the collective width (W2) of the struts (118) that define the commissure window (120) and the commissure window (120) itself.

2. The prosthetic device of claim 1, wherein the flexible connector (152) comprises a main body and first and second free edge portions (174b), and wherein the first and second free edge portions (174b) are folded around respective axially-extending struts (118) of the frame (102).

3. The prosthetic device of any one of claims 1-2, further comprising a wedge member (180) disposed on a radially inner facing surface of the flexible connector (152) between the adjacent leaflet tabs (114).

4. The prosthetic device of claim 3, wherein the wedge member (180) comprises a non-metallic material.

5. The prosthetic device of claim 3, wherein the wedge member (180) comprises a strip of PET fabric.

6. The prosthetic device of any one of claims 1-5, wherein the one or more primary sutures (168) are disposed adjacent a radially outer facing surface of the frame (102).

7. The prosthetic device of any one of claims 1-5, wherein the one or more primary sutures (168) are disposed adjacent a radially inner facing surface of the frame (102).

8. The prosthetic device of any one of claims 1-7, wherein the commissure windows (120) have an elongated rectangular shape.

9. The prosthetic device of any one of claims 1-8, wherein the flexible connector (152) has a rectangular shape;
preferably wherein the flexible connector (152) comprises an opening (308) extending through a thickness of a main body of the flexible connector (152);
more preferably wherein the opening (308) is sized such that a wedge member (180) can be disposed through the opening (308).

10. The prosthetic device of any one of claims 1-9, wherein the one or more secondary sutures (182) comprise a first secondary suture (182a) and a second secondary suture (182b), wherein the first secondary suture (182a) extends through the radially outer layer (184) of the flexible connector (152), a first commissure tab (144a) of the one or more commissure tabs (144), a first radially inner layer (186a) of the flexible connector (152), and a first free edge portion (174a) of the flexible connector (152), and wherein the second secondary suture (182b) extends through the radially outer layer (184) of the flexible connector (152), a second commissure tab (144b) of the one or more commissure tabs (144), a second radially inner layer (186b) of the flexible connector (152), and a second free edge portion (174b) of the flexible connector (152);
preferably further comprising a wedge member (180) disposed on a radially inner facing surface of the flexible connector (152) between the first and second commissure tabs (144b), wherein the first and second secondary sutures (182b) each extend through the wedge member (180).

11. The prosthetic device of any one of claims 1-9, wherein the one or more secondary sutures (182) comprise a secondary suture (182) that extends in a figure-eight pattern.

12. The prosthetic device of claim 11, wherein the figure-eight pattern extends through a radially outer layer (184) of the flexible connector (152), a first commissure tab (144a), a first radially inner layer (186a) of the flexible connector (152), a first free edge portion (174a) of the flexible connector (152), a second commissure tab (144b), a second radially inner layer (186b) of the flexible connector (152), and a second free edge portion (174b) of the flexible connector (152).

13. The prosthetic device of claim 12, wherein the commissure (132) further comprises a wedge member (180) disposed on a radially inner surface of the flexible connector (152), and wherein the figure-eight pattern passes through the wedge member (180) twice.

14. The prosthetic device of any one of claims 11-13, wherein the figure-eight pattern is a first figure-eight pattern (200a), and wherein the secondary suture (182) is sewn in a plurality of figure-eight patterns extending axially along a length of the commissure (132);
preferably wherein each figure-eight pattern is connected to one or more adjacent figure-eight patterns via one or more axially-extending portions of the secondary suture (182);
more preferably wherein the axially-extending portions of the secondary suture (182) are disposed on alternating sides of the wedge member (180).

15. The prosthetic device of any one of claims 1-14, wherein an outflow edge (158) of each of the one or more commissure tabs (144) is disposed at an angle relative to a longitudinal axis (A) of the leaflet (114);
preferably wherein the angle is a 90 degree angle.

## Patentansprüche

1. Implantierbare Prothesenvorrichtung, umfassend:
einen radial expandierbaren und komprimierbaren ringförmigen Rahmen (102), der einen Einströmendabschnitt (110) und einen Ausströmendabschnitt (112) umfasst, wobei der Rahmen (102) eine oder mehrere sich axial erstreckende Streben (118) umfasst, die ein oder mehrere Kommissurfenster (120) definieren;
eine Klappenstruktur (104), die eine Vielzahl von Segeln (114) umfasst, die innerhalb des Rahmens (102) angeordnet sind, wobei jedes Segel (114) einen Hauptkörper und eine oder mehrere Kommissurlaschen (144) umfasst;
eine Kommissur (132), die ein flexibles Verbindungselement (152) und die Kommissurlaschen (144) von zwei benachbarten Segeln (114) umfasst, die über eine oder mehrere Primärnähte (168) mit dem flexiblen Verbindungselement (152) gekoppelt sind; und
wobei die Kommissur (132) an einem jeweiligen Kommissurfenster (120) über eine oder mehrere Sekundärnähte (182) befestigt ist, wobei sich jede Sekundärnaht (182) durch eine radial äußere Schicht (184) des flexiblen Verbindungselements (152), eine jeweilige Kommissurlasche (144), eine radial innere Schicht (186) des flexiblen Verbindungselements (152) und einen freien Randabschnitt (174) des flexiblen Verbindungselements (152) erstreckt; und wobei:
eine Gesamtbreite (W1) der Kommissur (132) gleich oder kleiner ist als eine kombinierte Breite (W2) der einen oder mehreren sich axial erstreckenden Streben (118) und des Kommissurfensters (120); oder
die Kommissurlaschen (144) so bemessen sind, dass sie, wenn sie gegen eine radial nach außen weisende Oberfläche des Rahmens (102) gefaltet sind, eine Breite (W1) aufweisen, die im Wesentlichen gleich oder kleiner ist als die kombinierte Breite (W2) der Streben (118), die das Kommissurfenster (120) definieren, und des Kommissurfensters (120) selbst.

2. Prothesenvorrichtung gemäß Anspruch 1, wobei das flexible Verbindungselement (152) einen Hauptkörper und erste und zweite freie Randabschnitte (174b) umfasst, und wobei die ersten und die zweiten freien Randabschnitte (174b) um jeweilige sich axial erstreckende Streben (118) des Rahmens (102) gefaltet sind.

3. Prothesenvorrichtung gemäß einem der Ansprüche 1-2, ferner umfassend ein Keilelement (180), das auf einer radial nach innen weisenden Oberfläche des flexiblen Verbindungselements (152) zwischen den benachbarten Segellaschen (114) angeordnet ist.

4. Prothesenvorrichtung gemäß Anspruch 3, wobei das Keilelement (180) ein nichtmetallisches Material umfasst.

5. Prothesenvorrichtung gemäß Anspruch 3, wobei das Keilelement (180) einen Streifen aus PET-Gewebe umfasst.

6. Prothesenvorrichtung gemäß einem der Ansprüche 1-5, wobei die eine oder mehreren Primärnähte (168) benachbart zu einer radial nach außen weisenden Oberfläche des Rahmens (102) angeordnet sind.

7. Prothesenvorrichtung gemäß einem der Ansprüche 1-5, wobei die eine oder mehreren Primärnähte (168) benachbart zu einer radial nach innen weisenden Oberfläche des Rahmens (102) angeordnet sind.

8. Prothesenvorrichtung gemäß einem der Ansprüche 1-7, wobei die Kommissurfenster (120) eine länglich rechteckige Form aufweisen.

9. Prothesenvorrichtung gemäß einem der Ansprüche 1-8, wobei das flexible Verbindungselement (152) eine rechteckige Form aufweist;
vorzugsweise wobei das flexible Verbindungselement (152) eine Öffnung (308) umfasst, die sich durch eine Dicke eines Hauptkörpers des flexiblen Verbindungselements (152) erstreckt;
mehr bevorzugt wobei die Öffnung (308) so bemessen ist, dass ein Keilelement (180) durch die Öffnung (308) angeordnet werden kann.

10. Prothesenvorrichtung gemäß einem der Ansprüche 1-9, wobei die eine oder mehreren Sekundärnähte (182) eine erste Sekundärnaht (182a) und eine zweite Sekundärnaht (182b) umfassen, wobei sich die erste Sekundärnaht (182a) durch die radial äußere Schicht (184) des flexiblen Verbindungselements (152), eine erste Kommissurlasche (144a) der einen oder mehreren Kommissurlaschen (144), eine erste radial innere Schicht (186a) des flexiblen Verbindungselements (152) und einen ersten freien Randabschnitt (174a) des flexiblen Verbindungselements (152) erstreckt, und wobei sich die zweite Sekundärnaht (182b) durch die radial äußere Schicht (184) des flexiblen Verbindungselements (152), eine zweite Kommissurlasche (144b) der einen oder mehreren Kommissurlaschen (144), eine zweite radial innere Schicht (186b) des flexiblen Verbindungselements (152) und einen zweiten freien Randabschnitt (174b) des flexiblen Verbindungselements (152) erstreckt;
vorzugsweise ferner umfassend ein Keilelement (180), das auf einer radial nach innen weisenden Oberfläche des flexiblen Verbindungselements (152) zwischen der ersten und der zweiten Kommissurlasche (144b) angeordnet ist, wobei sich die erste und die zweite Sekundärnaht (182b) jeweils durch das Keilelement (180) erstrecken.

11. Prothesenvorrichtung gemäß einem der Ansprüche 1-9, wobei die eine oder mehreren Sekundärnähte (182) eine Sekundärnaht (182) umfassen, die sich in einem Achtermuster erstreckt.

12. Prothesenvorrichtung gemäß Anspruch 11, wobei sich das Achtermuster durch eine radial äußere Schicht (184) des flexiblen Verbindungselements (152), eine erste Kommissurlasche (144a), eine erste radial innere Schicht (186a) des flexiblen Verbindungselements (152), einen ersten freien Randabschnitt (174a) des flexiblen Verbindungselements (152), eine zweite Kommissurlasche (144b), eine zweite radial innere Schicht (186b) des flexiblen Verbindungselements (152) und einen zweiten freien Randabschnitt (174b) des flexiblen Verbindungselements (152) erstreckt.

13. Prothesenvorrichtung gemäß Anspruch 12, wobei die Kommissur (132) ferner ein Keilelement (180) umfasst, das auf einer radial inneren Oberfläche des flexiblen Verbindungselements (152) angeordnet ist, und wobei das Achtermuster zweimal durch das Keilelement (180) verläuft.

14. Prothesenvorrichtung gemäß einem der Ansprüche 11-13, wobei das Achtermuster ein erstes Achtermuster (200a) ist, und wobei die Sekundärnaht (182) in einer Vielzahl von Achtermustern, die sich axial entlang einer Länge der Kommissur (132) erstrecken, vernäht ist;
vorzugsweise wobei jedes Achtermuster über einen oder mehrere sich axial erstreckende Abschnitte der Sekundärnaht (182) mit einem oder mehreren benachbarten Achtermustern verbunden ist;
mehr bevorzugt wobei die sich axial erstreckenden Abschnitte der Sekundärnaht (182) auf wechselnden Seiten des Keilelements (180) angeordnet sind.

15. Prothesenvorrichtung gemäß einem der Ansprüche 1-14, wobei eine Ausströmkante (158) jeder der einen oder mehreren Kommissurlaschen (144) in einem Winkel relativ zu einer Längsachse (A) des Segels (114) angeordnet ist;
vorzugsweise wobei der Winkel ein 90-Grad-Winkel ist.

## Revendications

1. Dispositif prothétique implantable comprenant :
un cadre (102) annulaire radialement déployable et compressible comprenant une partie extrémité d'entrée (110) et une partie extrémité de sortie (112), le cadre (102) comprenant une ou plusieurs entretoises (118) s'étendant axialement qui définissent une ou plusieurs fenêtres (120) de commissure ;
une structure valvulaire (104) comprenant une pluralité de feuillets (114) agencés à l'intérieur du cadre (102), chaque feuillet (114) comprenant un corps principal et une ou plusieurs languettes (144) de commissure ;
une commissure (132) comprenant un raccord flexible (152) et les languettes (144) de commissure de deux feuillets adjacents (114) accouplés au raccord flexible (152) par l'intermédiaire d'une ou de plusieurs sutures primaires (168) ; et
la commissure (132) étant fixée fermement à une fenêtre (120) de commissure respective par l'intermédiaire d'une ou de plusieurs sutures secondaires (182), chaque suture secondaire (182) s'étendant à travers une couche radialement externe (184) du raccord flexible (152), une languette (144) de commissure respective, une couche radialement interne (186) du raccord flexible (152) et une partie de bord libre (174) du raccord flexible (152) ; et
une largeur globale (W1) de la commissure (132) étant égale ou inférieure à une largeur collective (W2) de ladite une ou desdites plusieurs entretoises (118) s'étendant axialement et de la fenêtre (120) de commissure ; ou
les languettes (144) de commissure étant dimensionnées de telle sorte que, lorsqu'elles sont pliées contre une surface tournée radialement vers l'extérieur du cadre (102), elles présentent une largeur (W1) qui est sensiblement égale ou inférieure à la largeur collective (W2) des entretoises (118) qui définissent la fenêtre (120) de commissure et de la fenêtre (120) de commissure elle-même.

2. Dispositif prothétique selon la revendication 1, le raccord flexible (152) comprenant un corps principal et des première et seconde parties de bord libre (174b) et les première et seconde parties de bord libre (174b) étant pliées autour d'entretoises (118) s'étendant axialement respectives du cadre (102).

3. Dispositif prothétique selon l'une quelconque des revendications 1 à 2, comprenant en outre un élément de coin (180) disposé sur une surface tournée radialement vers l'intérieur du raccord flexible (152) entre les languettes (114) de feuillet adjacentes.

4. Dispositif prothétique selon la revendication 3, l'élément de coin (180) comprenant un matériau non métallique.

5. Dispositif prothétique selon la revendication 3, l'élément de coin (180) comprenant une bande de tissu en PET.

6. Dispositif prothétique selon l'une quelconque des revendications 1 à 5, ladite une ou lesdites plusieurs sutures primaires (168) étant disposées de manière adjacente à une surface tournée radialement vers l'extérieur du cadre (102).

7. Dispositif prothétique selon l'une quelconque des revendications 1 à 5, ladite une ou lesdites plusieurs sutures primaires (168) étant disposées de manière adjacente à une surface tournée radialement vers l'intérieur du cadre (102).

8. Dispositif prothétique selon l'une quelconque des revendications 1 à 7, les fenêtres (120) de commissure présentant une forme rectangulaire allongée.

9. Dispositif prothétique selon l'une quelconque des revendications 1 à 8, le raccord flexible (152) présentant une forme rectangulaire ;
de préférence, le raccord flexible (152) comprenant une ouverture (308) s'étendant à travers une épaisseur d'un corps principal du raccord flexible (152) ;
plus préférablement, l'ouverture (308) étant dimensionnée de telle sorte qu'un élément de coin (180) peut être disposé à travers l'ouverture (308).

10. Dispositif prothétique selon l'une quelconque des revendications 1 à 9, ladite une ou lesdites plusieurs sutures secondaires (182) comprenant une première suture secondaire (182a) et une seconde suture secondaire (182b), la première suture secondaire (182a) s'étendant à travers la couche radialement externe (184) du raccord flexible (152), une première languette (144a) de commissure parmi ladite une ou lesdites plusieurs languettes (144) de commissure, une première couche radialement interne (186a) du raccord flexible (152) et une première partie de bord libre (174a) du raccord flexible (152) et la seconde suture secondaire (182b) s'étendant à travers la couche radialement externe (184) du raccord flexible (152), une seconde languette (144b) de commissure parmi ladite une ou lesdites plusieurs languettes (144) de commissure, une seconde couche radialement interne (186b) du raccord flexible (152) et une seconde partie de bord libre (174b) du raccord flexible (152) ;
de préférence, comprenant en outre un élément de coin (180) disposé sur une surface tournée radialement vers l'intérieur du raccord flexible (152) entre les première et seconde languettes (144b) de commissure, les première et seconde sutures secondaires (182b) s'étendant chacune à travers l'élément de coin (180).

11. Dispositif prothétique selon l'une quelconque des revendications 1 à 9, ladite une ou lesdites plusieurs sutures secondaires (182) comprenant une suture secondaire (182) qui s'étend selon un motif en forme de huit.

12. Dispositif prothétique selon la revendication 11, le motif en forme de huit s'étendant à travers une couche radialement externe (184) du raccord flexible (152), une première languette (144a) de commissure, une première couche radialement interne (186a) du raccord flexible (152), une première partie de bord libre (174a) du raccord flexible (152), une seconde languette (144b) de commissure, une seconde couche radialement interne (186b) du raccord flexible (152) et une seconde partie de bord libre (174b) du raccord flexible (152).

13. Dispositif prothétique selon la revendication 12, la commissure (132) comprenant en outre un élément de coin (180) disposé sur une surface radialement interne du raccord flexible (152) et le motif en forme de huit passant deux fois à travers l'élément de coin (180).

14. Dispositif prothétique selon l'une quelconque des revendications 11 à 13, le motif en forme de huit étant un premier motif en forme de huit (200a) et la suture secondaire (182) étant cousue en une pluralité de motifs en forme de huit s'étendant axialement le long d'une longueur de la commissure (132) ;
de préférence, chaque motif en forme de huit étant relié à un ou plusieurs motifs en forme de huit adjacents par l'intermédiaire d'une ou de plusieurs parties s'étendant axialement de la suture secondaire (182) ;
plus préférablement, les parties s'étendant axialement de la suture secondaire (182) étant disposées sur des côtés alternés de l'élément de coin (180).

15. Dispositif prothétique selon l'une quelconque des revendications 1 à 14, un bord de sortie (158) de chacune de ladite une ou desdites plusieurs languettes (144) de commissure étant disposé selon un angle par rapport à un axe longitudinal (A) du feuillet (114) ;
de préférence, l'angle étant un angle de 90 degrés.
